# EUROPEAN PATENT APPLICATION

(11) **EP 3 714 864 A1**
(43) Date of publication of application: **30.09.2020**
(21) Application number: 20163889.7
(22) Date of filing: 18.03.2020
(51) Int. Cl.: A61K 8/04, A61Q 5/06, A61K 8/19

(54) **NITROGEN PROPELLED HAIR STYLING COMPOSITION**

(30) Priority: 29.03.2019 US 201962826608 P
(71) Applicant: Henkel IP & Holding GmbH, 40589 Düsseldorf (DE)
(72) Inventor: Sharp, Carter Briggs, Playa Del Rey, 90293 (US); Stucky, Marc Andrew, Rancho Palos Verdes, 90275 (US); Lowenthal, Matthew, Playa Vista, 90094 (US); Schaeffler, Robert, Marina del Rey, 90292 (US)
(74) Representative: Henkel IP Department

(57) **Abstract**

Compositions and methods for the styling and temporary hold of keratin are provided. An exemplary composition for the styling and temporary hold of keratin includes a solvent, a polymer, and a propellant having nitrogen. After being dispensed from a compressed container, the composition has an increased density at ambient temperature and pressure as compared to conventional non-nitrogen propelled compositions. Further, such a composition may be free of volatile organic compounds (VOCs).

## Description

### FIELD OF THE INVENTION

The present disclosure generally relates to hair styling compositions, such as hair mousses, and more particularly relates to such hair styling compositions that are utilize nitrogen as a propellant.

### BACKGROUND OF THE INVENTION

An attractive-looking hairstyle is generally regarded these days as an indispensable element of a well-groomed appearance. Many fashionable hairstyles may be constructed, or maintained for a period of time of up to several days, only with the use of setting active agents. Temporary shaping results that are intended to result in good hold without impairing the healthy appearance of the hair can be achieved by using hair styling products such as mousse, hair spray, hair wax, hair gel, blow-dry waves, and the like.

Corresponding agents for temporary shaping usually contain synthetic polymers as a shaping component. Preparations that contain a dissolved or dispersed polymer can be applied onto the hair by means of propellant gases or using a pump mechanism. Hair mousses, hair gels and hair waxes in particular, however, are generally not applied directly onto the hair but instead are distributed in the hair using a comb or the hands.

The most important property of an agent for the temporary deformation of keratinic fibers, hereinafter also called a "styling agent," is to impart the strongest possible hold to the treated fibers in the shape that is generated. If the keratinic fibers involved are human hairs, terms also used are a strong "hairstyle hold" or a high "degree of hold" of the styling agent. The hairstyle hold is determined substantially by the nature and quantity of the setting polymer used, although the further constituents of the styling agent can also have an influence.

In addition to a high degree of hold, styling agents must meet a large number of further requirements. These can be subdivided roughly into: properties on the hair; properties of the particular formulation, i.e., the mousse, foam, gel, or sprayed aerosol; and properties that relate to the handling of the styling agent, the properties on the hair being of particular importance. Moisture resistance, low tack, and a balanced conditioning effect may be mentioned in particular. In addition, a styling agent should be universally usable for, if possible, all types of hair.

Hair mousse is a hair styling product for smoothing, straightening, curling, blow drying and generally styling hair that is typically provided as an aqueous mixture in a container under pressure from a volatile organic compound (VOC) propellant, such as 152A, and, upon release from the container, forms a fluffy foam. Conventional high hold mousses are limited by stickiness and workability.

It is desirable to provide a hair mousse product that is capable of providing excellent hold and style memory that lasts for up to four days. In addition it is desirable to provide such a hair mousse product with little or no VOC content. Furthermore, other desirable features and characteristics will become apparent from the subsequent detailed description and the appended claims, taken in conjunction with the accompanying drawings and the foregoing technical field and background.

### BRIEF SUMMARY OF THE INVENTION

Compositions and methods for the styling and temporary hold of keratin are provided. An exemplary composition for the styling and temporary hold of keratin includes a solvent, a polymer, and a propellant comprising nitrogen. After being dispensed from a compressed container, the composition has an increased density at ambient temperature and pressure as compared to conventional non-nitrogen propelled compositions. Further, such a composition may be free of volatile organic compounds (VOCs).

In an exemplary embodiment, a method for styling hair includes releasing a composition from a container under pressure, the composition comprising a solvent, a polymer, and a propellant comprising nitrogen. Further, the method includes applying the composition to the hair and styling the hair as desired.

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

### BRIEF DESCRIPTION OF THE DRAWINGS

Embodiments of compositions and methods for the styling and temporary hold of keratin will hereinafter be described in conjunction with the following drawing figures, wherein like numerals denote like elements, and wherein:
FIG. 1 illustrates the texture of the exemplary nitrogen-based formulation directly after being released from a compressed container and of a comparative non-nitrogen-based formulation directly after being released from a compressed container.
FIG. 2 shows images of a subject's hair following the application, on the left side of the hair, of a composition according to embodiments herein and, on the right side of the hair, of a comparative composition using a conventional propellant and styling of the hair, directly following styling, a day after styling, and two days after styling.

### DETAILED DESCRIPTION OF THE INVENTION

The following detailed description is merely exemplary in nature and is not intended to limit the compositions and methods for the styling and temporary hold of keratin as claimed herein. Furthermore, there is no intention to be bound by any expressed or implied theory presented in the preceding technical field, background or brief summary, or in the following detailed description. As used herein, "keratinic fibers" are understood in principle as all animal hairs, e.g., wool, horsehair, angora hair, furs, feathers, and products or textiles produced therefrom. In exemplary embodiments, the keratinic fibers are human hairs.

An exemplary embodiment described herein is related to a hair styling product for smoothing, straightening, curling, blow drying and generally styling hair. The product is capable of providing excelling hold and style memory that lasts for up to four days. An exemplary embodiment is a nitrogen-based mousse provided with a revolutionary smooth, workable application with an ultra-high hold. The product provides for a creamy, conditioning foam allowing for higher hold with less stickiness and increased volume.

Traditional mousse styling products have a fluffy foam consistency. These products use traditional propellants, such as 1,1-difluoroethane (HFC-152a), commonly referred to as 152a. On the other hand, exemplary embodiments herein utilize nitrogen as a propellant for styling product. When nitrogen is used as the propellant, the styling product is provided with a creme like consistency unlike a typical mousse, which is typically a foam. Without being limited by theory, it is believed that the creme consistency is due to the nitrogen propellant producing smaller gas pockets after being dispensed, as compared to conventional propellant. As a result, the exemplary styling product has a more even application than a standard mousse. Due to this, the product holds the hair in a style for a longer period of time. Hold is due to the capillary forces between two pieces of adjacent hair fibers. Having smaller pockets allows for the formation of more capillary bridges between the two hair fibers, resulting in a more flexible and harder hold. The formulation of exemplary embodiments is provided with increased style memory or longevity and stronger hold. The product has a creamy consistency unlike the foam consistency of traditional mousse products.

As described herein, a hair styling composition, such as a hair mousse, is provided with a propellant comprising nitrogen. Such nitrogen based mousses may consist of a propellant consisting of nitrogen, a propellant consisting essentially of nitrogen, or a propellant comprising nitrogen. For example, the propellant may be 100 weight percent (wt.%) nitrogen. In exemplary embodiments, the composition is free of volatile organic compounds (VOCs). In other embodiments, the composition has a volatile organic compound (VOC) content of less than 1 weight percent (wt.%) based on the total weight of the composition, such as less than 0.75 wt. %, for example less than 0.5 wt. %, such as less than 0.25 wt. %, for example less than 0.1 wt. %, such as less than 0.5 wt.%, based on the total weight of the composition. As defined by the EPA, volatile organic compounds (VOCs) include any chemical substance which contains a compound of carbon, excluding carbon monoxide, carbon dioxide, carbonic acid, metallic carbides or carbonates, and ammonium carbonate, determined to have non-negligible photochemical reactivity. Organic compounds that are exempted from the category of VOCs by the EPA based on having negligible photochemical reactivity, (exempted organic compounds (EOCs) include methane; ethane; methylene chloride (dichloromethane); 1,1,1-trichloroethane (methyl chloroform); 1,1,2-trichloro-1,2,2- trifluoroethane (CFC-113); trichlorofluoromethane (CFC-11); dichlorodifluoromethane (CFC-12); chlorodifluoromethane (HCFC-22); trifluoromethane (HFC-23); 1,2-dichloro 1,1,2,2-tetrafluoroethane (CFC-114); chloropentafluoroethane (CFC-115); 1,1,1-trifluoro 2,2-dichloroethane (HCFC-123); 1,1,1,2-tetrafluoroethane (HFC-134a); 1,1-dichloro 1-fluoroethane (HCFC-141b); 1-chloro 1,1-difluoroethane (HCFC-142b); 2-chloro-1,1,1,2-tetrafluoroethane (HCFC-124); pentafluoroethane (HFC-125); 1,1,2,2-tetrafluoroethane (HFC-134); 1,1,1-trifluoroethane (HFC-143a); 1,1-difluoroethane (HFC-152a); parachlorobenzotrifluoride (PCBTF); cyclic, branched, or linear completely methylated siloxanes; acetone; perchloroethylene (tetrachloroethylene); 3,3-dichloro1,1,1,2,2-pentafluoropropane (HCFC-225ca); 1,3-dichloro-1,1,2,2,3- pentafluoropropane (HCFC-225cb); 1,1,1,2,3,4,4,5,5,5-decafluoropentane (HFC 43- 10mee); difluoromethane (HFC-32); ethylfluoride (HFC-161); 1,1,1,3,3,3- hexafluoropropane (HFC-236fa); 1,1,2,2,3-pentafluoropropane (HFC-245ca); 1,1,2,3,3-pentafluoropropane (HFC-245ea); 1,1,1,2,3-pentafluoropropane (HFC- 245eb); 1,1,1,3,3-pentafluoropropane (HFC-245fa); 1,1,1,2,3,3-hexafluoropropane (HFC-236ea); 1,1,1,3,3-pentafluorobutane (HFC-365mfc); chlorofluoromethane (HCFC-31); 1 chloro-1-fluoroethane (HCFC-151a); 1,2-dichloro-1,1,2- trifluoroethane (HCFC-123a); 1,1,1,2,2,3,3,4,4-nonafluoro-4-methoxy-butane (C4F9OCH3or HFE-7100); 2-(difluoromethoxymethyl)-1,1,1,2,3,3,3-heptafluoropropane ((CF3)2CFCF2OCH3); 1-ethoxy-1,1,2,2,3,3,4,4,4- nonafluorobutane (C4F9OC2H5or HFE-7200); 2-(ethoxydifluoromethyl)- 1,1,1,2,3,3,3-heptafluoropropane ((CF3)2CFCF2OC2H5); methyl acetate, 1,1,1,2,2,3,3-heptafluoro-3-methoxy-propane (n-C3F7OCH3, HFE-7000), 3-ethoxy 1,1,1,2,3,4,4,5,5,6,6,6-dodecafluoro-2-(trifluoromethyl) hexane (HFE-7500), 1,1,1,2,3,3,3-heptafluoropropane (HFC 227ea), methyl formate (HCOOCH3), (1) 1,1,1,2,2,3,4,5,5,5-decafluoro-3-methoxy-4-trifluoromethyl-pentane (HFE-7300); propylene carbonate; dimethyl carbonate; and perfluorocarbon compounds which fall into these classes: 2 (i) Cyclic, branched, or linear, completely fluorinated alkanes; (ii) Cyclic, branched, or linear, completely fluorinated ethers with no unsaturations; (iii) Cyclic, branched, or linear, completely fluorinated tertiary amines with no unsaturations; and (iv) Sulfur containing perfluorocarbons with no unsaturations and with sulfur bonds only to carbon and fluorine. In exemplary embodiments, the composition is free of the above listed exempted organic compounds (EOCs). In other embodiments, the composition has an EOC content of less than 1 weight percent (wt.%) based on the total weight of the composition, such as less than 0.75 wt. %, for example less than 0.5 wt. %, such as less than 0.25 wt. %, for example less than 0.1 wt. %, such as less than 0.5 wt.%, based on the total weight of the composition.

In exemplary embodiments, the composition comprises less than 1.1 weight percent (wt.%) of the propellant based on the total weight of the composition. For example, the composition may comprise less than 1.0 wt.%, such as less than 0.95 wt.%, for example less than 0.9 wt.%, such as less than 0.85 wt.%, for example less than 0.8 wt.%, such as less than 0.75 wt.%, for example less than 0.7 wt.%, such as less than 0.65 wt.%, for example less than 0.6 wt.%, such as less than 0.55 wt.%, for example less than 0.5 wt.%, such as less than 0.45 wt.%, for example less than 0.4 wt.%, of the propellant based on the total weight of the composition.

In exemplary embodiments, the composition comprises more than 0.1 wt.% of the propellant based on the total weight of the composition. For example, the composition may comprise more than 0.15 wt.%, such as more than 0.2 wt.%, for example more than 0.25 wt.%, such as more than 0.3 wt.%, for example more than 0.35 wt.%, such as more than 0.4 wt.%, for example more than 0.45 wt.%, such as more than 0.5 wt.%, for example more than 0.55 wt.%, such as more than 0.6 wt.%, for example more than 0.65 wt.%, such as more than 0.7 wt.%, of the propellant based on the total weight of the composition. In certain embodiments, the composition comprises from about 0.4 to about 0.8 wt.% of propellant based on the total weight of the composition.

In addition to the nitrogen propellant, an exemplary composition for the styling and temporary hold of keratin includes a solvent and a polymer. The solvent and polymer may include any of the known solvents and polymers suitable for use in personal care, and specifically for hair care.

In an exemplary embodiment, the exemplary composition includes the components listed in Table 1:

**TABLE 1: Example Formulation**

| **Trade Name** | **INCI** | **Wt. %** | **Function** |
|---|---|---|---|
| Water Purified USP | Water (Aqua) | 87.07 | Diluent/Solvent |
| Panthenol | Panthenol | 0.2 | Strengthener |
| Dehyquart A CA | Cetrimonium Chloride | 0.21 | Detangler |
| | Water (Aqua) | 0.49 | Diluent/Solvent |
| PVP K-30 | PVP | 3.0 | Fixative |
| Glycerine - USP/Kosher 99.7% Min | Glycerin | 0.75 | Emollient |
| Cetearyl Alcohol | Cetearyl Alcohol | 2.0 | Emulsifier |
| White Protopet 1S Petrolatum | Petrolatum | 0.5 | Emollient |
| Drakeol 9 LT Min Oil NF | Mineral Oil | 0.5 | Shine |
| Incroquat TMC-85 | Behentrimonium Chloride | 0.34 | Conditioner |
| | Isopropyl Alcohol | 0.06 | Solvent |
| Versene NA2 Crystals | Disodium EDTA | 0.01 | Chelating Agent |
| Germall plus | Diazolidinyl Urea | 0.069125 | Preservative |
| | Iodopropynyl Butylcarbamate | 0.000875 | Preservative |
| 10084 12 Nitro Mousse Fragrance MOD A | Fragrance | 0.2359 | Fragrance |
| | Linalool | 0.006 | Fragrance Allergen |
| | Coumarin | 0.002 | Fragrance Allergen |
| | Eugenol | 0.0015 | Fragrance Allergen |
| | Benzyl Salicylate | 0.0246 | Fragrance Allergen |
| | Hexyl Cinnamal | 0.03 | Fragrance Allergen |
| Dow Corning CE-8170 AF microemulsion | Water (Aqua) | 0.54 | Diluent/Solvent |
| | Amodimethicone | 0.2 | Frizz Control |
| | C11-15 Pareth-7 | 0.07 | Emulsifier |
| | Laureth -9 | 0.07 | Emulsifier |
| | Glycerin | 0.05 | Emollient |
| | Trideceth-12 | 0.05 | Emulsifier |
| | Phenoxyethanol | 0.01 | Preservative |
| | Acetic Acid | 0.01 | Preservative |
| PVP/VA W-735 | PVP/VA Copolymer | 1.0 | Fixative |
| | Water | 1.0 | Diluent/Solvent |
| Luviquat PQ ATI | Polyquaternium-11 | 0.2 | Fixative |
| | Water (Aqua) | 0.7925 | Diluent/Solvent |
| | Phenoxyethanol | 0.00675 | Preservative |
| | Ethylhexylglycerin | 0.00075 | Preservative Booster |
| Nitrogen | Nitrogen | 0.5 | Compressed Gas |

The composition may include additional or fewer components as is desired.

An exemplary composition including the components of Table 1 was compared to an otherwise identical product that utilized 152A propellant rather than nitrogen. The comparison tested hold and curl hold. For example, a MTT175 3-Point Bend (Hold) test was performed.

The MTT175 3-Point Bend (Hold) test measures the hold a product gives to the hair. In the test, the exemplary nitrogen-based formulation and the comparative 152A propellant formulation were applied to hair swatches, which were then placed on hair tress on a machine. The amount of force required to break the hold in the hair is measured. The greater the force, the greater the hold provides to the hair. The test not only measures hold, but also rebound or flexibility.

The hold level was evaluated by using MTT175 instrument. The exemplary nitrogen-based formulation was found to require 0.0062 joules of force to break the hold, and had a hold level rank of 10. The comparative 152A propellant formulation was found to require 0.0039 joules of force to break the hold, and had a hold level rank of 8.

Thus, the exemplary nitrogen-based formulation provided the hair with stronger hold than the comparative 152A propellant formulation. The results indicate that the exemplary nitrogen-based formulation treated swatch required greater amount of force to break the hold than the comparative 152A propellant formulation treated swatch.

To evaluate the curl hold performance, the exemplary nitrogen based formulation and the comparative 152A propellant formulation were applied to damp, level 8 straight hair swatches. Then after blow drying, the hair was curled, and the treated samples were hanged at room temperature for four days. The curl length was measured daily over the four day period.

For the exemplary nitrogen based formulation, the initial swatch curl length was 4.75 inches, and the length after four days was 5.50 inches. Thus, the length increased by 16% over four days. For the comparative 152A propellant formulation, the initial swatch curl length was 4.50 inches, and the length after four days was 5.75 inches. Thus, the length increased by 28% over four days. The larger the percent change from the initial measurement, the more curl stretch and the less curl retention was shown. The results indicate that the percent change of the exemplary nitrogen based formulation is less than that of the comparative 152A propellant formulation. Therefore, the samples treated with the exemplary nitrogen based formulation provide exhibited better retained curls.

In summary, the hold and curl testing illustrated that the exemplary nitrogen based formulation provides a stronger hold than the comparative 152A propellant formulation. Further, the exemplary nitrogen based formulation provides better curl retention than the comparative 152A propellant formulation.

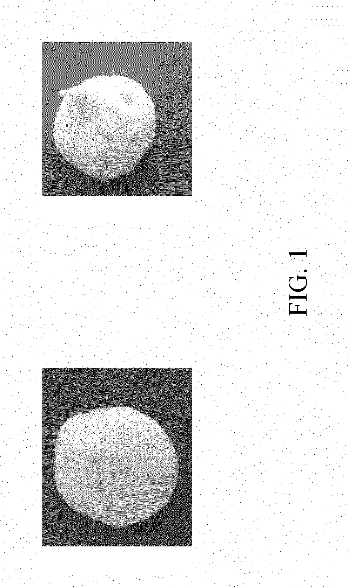

FIG. 1 is an image of a sample of the exemplary nitrogen based formulation (left) and the comparative 152A propellant formulation (right) directly after being released from compressed containers, such as one minute after being released. As shown, the exemplary nitrogen based formulation is denser and has a creamy texture and the comparative 152A propellant formulation is less dense and more foam like.

After being released from pressure, the exemplary composition forms nitrogen gas pockets within the composition. As has been discovered, the nitrogen gas pockets are smaller than those formed by a comparative conventional propellant. As a result, the exemplary composition is provided with an increased density as compared to conventional formations with larger gas pockets. For example, when applying the composition to the hair, i.e., directly after releasing the composition from the container, such as from one second to one minute after release, the composition has a density of greater than 0.25 g/mL at ambient temperature and pressure, such as greater than 0.3 g/mL, for example greater than 0.35 g/mL, such as greater than 0.4 g/mL, for example greater than 0.45 g/mL, such as greater than 0.5 g/mL, for example greater than 0.55 g/mL, such as greater than 0.6 g/mL, for example greater than 0.65 g/mL, such as greater than 0.7 g/mL, for example greater than 0.75 g/mL, such as greater than 0.8 g/mL, for example greater than 0.85 g/mL, such as greater than 0.9 g/mL, for example greater than 0.95 g/mL, such as greater than 1.0 g/mL, at ambient temperature and pressure. In certain embodiments, when applying the composition to the hair the composition has a density of from about 0.8 to about 1.0 g/mL at ambient temperature and pressure.

In exemplary embodiments, when applying the composition to the hair, i.e., directly after releasing the composition from the container, such as from one second to one minute after release, the composition has a density of less than 5.0 g/mL at ambient temperature and pressure, such as less than 2.5 g/mL, for example less than 2.0 g/mL, such as less than 1.9 g/mL, for example less than 1.8 g/mL, such as less than 1.7 g/mL, for example less than 1.6 g/mL, such as less than 1.5 g/mL, for example less than 1.4 g/mL, such as less than 1.3 g/mL, for example less than 1.25 g/mL, such as less than 1.2 g/mL, for example less than 1.15 g/mL, such as less than 1.1 g/mL, for example less than 1.05 g/mL, such as less than 1.0 g/mL, for example less than 0.95 g/mL, such as less than 0.9 g/mL, for example less than 0.85 g/mL, at ambient temperature and pressure. In certain embodiments, when applying the composition to the hair the composition has a density of from about 0.8 to about 1.0 g/mL at ambient temperature and pressure.

Density is a measurement of how tightly materials are packed together. Density is a measure of mass per volume (m/v). Weight per unit of volume is used to measure the compactness of a substance. Below, the compactness of exemplary embodiments described herein, i.e., nitrogen-based formulations, are compared to comparative formulations using 152A instead of nitrogen as the propellant.

To measure the density of sample, a 10 mL sample of a formulation was placed in beaker and the weight of the sample was recorded. To calculate the density, the weight of the sample was divided by the volume of the sample (inside the beaker). For example, the weight of a nitrogen-based embodiment (8.3698 g) was divided by volume of the formulation (10 mL): (8.3698 g /10 mL). Thus, the density of the exemplary embodiment was 0.84 g/mL.

The density of a comparative example using 152A as the propellant was also measured using the same procedure. The weight of 10 mL of the comparative example was found to be 1.5310 g. Thus, the density of the comparative example was 0.15 g/mL. As can be seen, the exemplary embodiment has a density that is over five times greater than the comparative example using 152A propellant.

Also described herein is a method for styling hair. The method includes releasing a composition from a container under pressure. The composition comprises a solvent, a polymer, and a propellant comprising nitrogen. The method includes, after releasing the composition from pressure, applying the composition to the hair and styling the hair as desired.

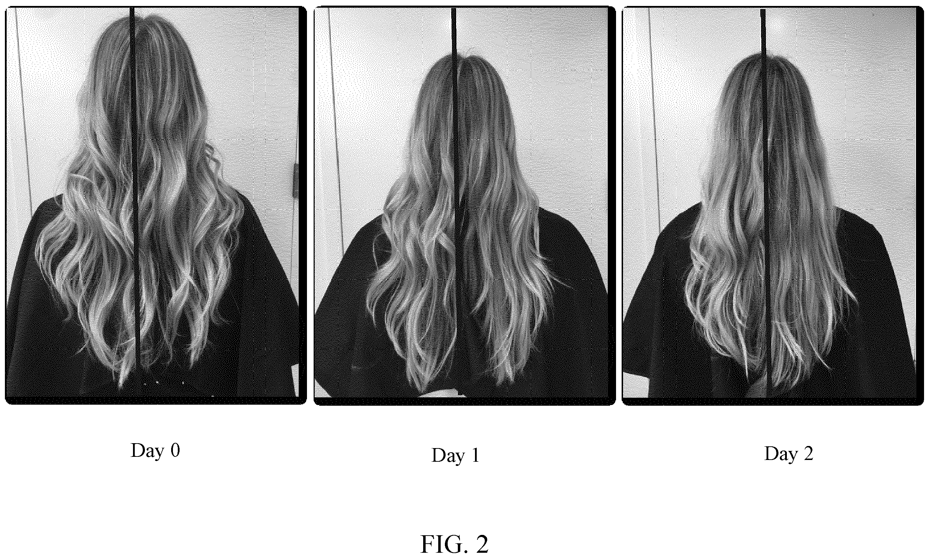

FIG. 2 shows images of a subject's hair following the application, on the left side of the hair, of a composition according to embodiments herein and, on the right side of the hair, of a comparative composition using a conventional propellant and styling of the hair, directly following styling (Day 0), a day after styling (Day 1), and two days after styling (Day 2). As may be seen, the curl hold of the composition using nitrogen as the propellant is greater at each time interval.

Testing was also performed for various compositions including nitrogen as a propellant (Rounds 1-5) and for a comparative composition using 152A as the propellant (Round 6). The amount of nitrogen is the same for each of Rounds 1-5, while the amount of polymer varies in Rounds 1-5. In each round, the product was shaken for thirty seconds before dispensing. Table 2 provides the results of testing.

**TABLE 2: Product Testing Results**

| | **Application** | **Fragrance** | **Ease of Use** | **End Result** | **Feel in Hair** | **Approved** |
|---|---|---|---|---|---|---|
| Round 1 | 1.0 | 1.5 | 1.0 | 3.0 | 4.0 | No |
| Round 2 | 1.0 | 4.5 | 1.0 | 3.0 | 3.5 | No |
| Round 3 | 4.5 | 4.5 | 3.0 | 4.5 | 4.5 | No |
| Round 4 | 1.0 | 4.5 | 3.0 | 1.5 | 1.5 | No |
| Round 5 | 5.0 | 4.5 | 4.5 | 5.0 | 4.5 | Yes |
| Round 6 Comparative 152A Composition | 4.0 | 4.5 | 3.0 | 4.0 | 4.0 | No |

Testing was done via a survey of a panel of users. Specifically, 10 to 15 users were provided with each sample of Rounds 1-6 in a pressurized container. Each user then released a portion of each sample, applied it to hair, and evaluated the portion in five categories using a scale of 1 to 5, with 1 being unacceptable and 5 being most desirable. Each user further provided a general grade of approved or not approved for use.

As can be seen, the composition of Round 1 received: a score of 1.0 for application, with notes that the composition was too wet; a score of 1.5 for fragrance, with notes that the fragrance turned with heat; a score of 1.0 for ease of use, with notes that the composition failed to dispense well; a score of 3.0 for end result, with notes that the composition provided too little volume to the hair; a score of 4.0 for feel in hair; and a grade of not approved for use.

The composition of Round 2 received: a score of 1.0 for application, with notes that the composition was too wet; a score of 4.5 for fragrance; a score of 1.0 for ease of use, with notes that the composition failed to dispense well; a score of 3.0 for end result, with notes that the composition provided too little volume to the hair; a score of 3.5 for feel in hair; and a grade of not approved for use.

The composition of Round 3 received: a score of 4.5 for application; a score of 4.5 for fragrance; a score of 3.0 for ease of use; a score of 4.5 for end result; a score of 4.5 for feel in hair; and a grade of not approved for use. Hold was graded as 15-17, on a scale of 1 (lowest) to 20 (highest).

The composition of Round 4 received: a score of 1.0 for application, with notes that the composition was difficult to work through hair; a score of 4.5 for fragrance; a score of 3.0 for ease of use; a score of 1.5 for end result, with notes that the composition provided too little volume to the hair; a score of 1.5 for feel in hair, with notes that the composition provided a gummy hold; and a grade of not approved for use.

The composition of Round 5 received: a score of 5.0 for application; a score of 4.5 for fragrance; a score of 4.5 for ease of use; a score of 5.0 for end result; a score of 4.5 for feel in hair; and a grade of approved for use. Hold was graded as 17-20, on a scale of 1 (lowest) to 20 (highest).

The comparative composition with 152A propellant of Round 6 received: a score of 4.0 for application; a score of 4.5 for fragrance; a score of 3.0 for ease of use, with notes that it was difficult to dispense; a score of 4.0 for end result; a score of 4.0 for feel in hair; and a grade of not approved for use.

While at least one exemplary embodiment has been presented in the foregoing detailed description, it should be appreciated that a vast number of variations exist. It should also be appreciated that the exemplary embodiment or embodiments described herein are not intended to limit the scope, applicability, or configuration of the claimed subject matter in any way. Rather, the foregoing detailed description will provide those skilled in the art with a convenient road map for implementing the described embodiment or embodiments. It should be understood that various changes can be made in the function and arrangement of elements without departing from the scope defined by the claims, which includes known equivalents and foreseeable equivalents at the time of filing this patent application.

## Claims

1. A composition for the styling and temporary hold of keratin, comprising:
a solvent,
a polymer, and
a propellant comprising nitrogen.

2. The composition of claim 1 wherein the composition is free of volatile organic compounds (VOCs).

3. The composition of claim 1 wherein the composition has a volatile organic compound (VOC) content of less than 1 weight percent (wt.%) based on the total weight of the composition.

4. The composition of claim 1 wherein the propellant consists essentially of nitrogen or wherein the propellant consists of nitrogen.

5. The composition of claim 1 wherein the composition comprises less than 0.8 weight percent (wt.%) or more than 0.4 weight percent (wt.%) of the propellant based on the total weight of the composition.

6. The composition of claim 1 wherein the composition comprises from about 0.4 to about 0.8 weight percent (wt.%) of the propellant based on the total weight of the composition.

7. The composition of claim 1 wherein, after being dispensed from a compressed container, the composition has a density of greater than 0.3 g/mL, preferable of greater than 0.5 g/mL, more preferably of greater than 0.7 g/mL at ambient temperature and pressure.

8. The composition of claim 1 wherein, after being dispensed from a compressed container, the composition has a density of from about 0.8 to about 1.0 g/mL at ambient temperature and pressure.

9. A method for styling hair, said method comprising:
releasing a composition from a container under pressure, the composition comprising a solvent, a polymer, and a propellant comprising nitrogen;
applying the composition to the hair;
styling the hair as desired.

10. The method of claim 9 wherein when applying the composition to the hair the composition has a density of greater than 0.3 g/mL, preferably of greater than 0.5 g/mL at ambient temperature and pressure.

11. The method of claim 9 wherein when applying the composition to the hair the composition has a density of greater than 0.7 g/mL at ambient temperature and pressure.

12. The method of claim 9 wherein when applying the composition to the hair the composition has a density of from about 0.8 to about 1.0 g/mL at ambient temperature and pressure.

13. The method of claim 9 wherein the composition is free of volatile organic compounds (VOCs).

14. The method of claim 9 wherein the propellant consists of nitrogen.

15. The method of claim 9 wherein the composition comprises less than 0.8 weight percent (wt.%) of the propellant based on the total weight of the composition.
